(19) Europäisches Patentamt
European Patent Office
Office européen des brevets

(11) Publication number: **0 272 609 B1**

(12) **EUROPEAN PATENT SPECIFICATION**

(45) Date of publication of patent specification: **11.03.92**

(51) Int. Cl.5: **C07H 21/04**, C07K 15/00

(21) Application number: **87118686.2**

(22) Date of filing: **16.12.87**

(54) **Amino acid sequence of human alpha2-plasmin inhibitor and cDNA and genomic DNA encoding said amino acid sequence.**

(30) Priority: **19.12.86 JP 301753/86**
**23.06.87 JP 154495/87**

(43) Date of publication of application:
**29.06.88 Bulletin 88/26**

(45) Publication of the grant of the patent:
**11.03.92 Bulletin 92/11**

(84) Designated Contracting States:
**CH DE FR GB LI**

(73) Proprietor: **TEIJIN LIMITED**
**11 Minami Honmachi 1-chome Higashi-ku**
**Osaka-shi Osaka-fu(JP)**

(72) Inventor: **Sumi, Yoshihiko**
**3-18-4, Tamadaira**
**Hino-shi Tokyo(JP)**
Inventor: **Ichikawa, Yataro**
**2-11-6, Kotesashi-cho**
**Tokorozawa-shi Saitama-ken(JP)**
Inventor: **Muramatsu, Masami**
**4-21-6, Kotesashi-cho**
**Tokorozawa-shi Saitama-ken(JP)**
Inventor: **Aoki, Nobuo**
**4-20-2-304, Hongo Bunkyo-ku**
**Tokyo(JP)**

(74) Representative: **Kraus, Walter, Dr. et al**
**Patentanwälte Kraus, Weisert & Partner**
**Thomas-Wimmer-Ring 15**
**W-8000 München 22(DE)**

EP 0 272 609 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relats to the amino acid sequence of a human $\alpha_2$-plasmin inhibitor (or $\alpha_2$-antiplasmin), and cDNA and genomic DNA encoding this sequence.

The human $\alpha_2$-plasmin inhibitor was first isolated and purified by Aoki and Moroi. It is known to be a strong plasmin inhibitor which instantaneously inhibits the esterase activity of plasmin, a fibrinolytic enzyme, and is a single-chain glycoprotein having a molecular weight of about 67,000 and containing 11.7% of sugar chains [M. Moroi & N. Aoki: The Journal of Biological Chemistry, 251, 5956-5965 (1976)].

On the other hand, it is known that the human $\alpha_2$-plasmin inhibitor has three types of active sites. One is a site of inhibiting the fibrinolytic action of plasmin (to be referred to sometimes as the "reactive site") [B. Wiman & D. Collen: The Journal of Biological Chemistry, 254, 9291-9297 (1979)]. A second is a site of binding to plasmin at the carboxyl terminal portion [B. Wiman & D. Collen: European Journal of Biochemistry, 84, 573-578 (1978)]. A third is a site of binding to fibrin at the amino terminal portion [Y. Sakata et al.: Thrombosis Research, 16, 279-282 (1979)].

It has already been determined that among the three acive sites of the human $\alpha_2$-plasmin inhibitor, the fibrin binding site is Gln which is the second amino acid from the amino terminus of the human $\alpha_2$-plasmin inhibitor [T. Tamaki & N. Aoki: The Journal of Biological Chemistry, 257, 14767-14772 (1982)]. A peptide fragment composed of 26 amino acids including the plasmin binding site is also known. It has been reported that this peptide fragment exists near the carboxy-terminus of the human $\alpha_2$-plasmin inhibitor [see T. Sasaki et al.: The Journal of Biochemistry, 99, 1699-1705 (1986)], but its position is not clear. It has been reported that the reactive site of the human $\alpha_2$-plasmin inhibitor is Leu-Met [see B. Wiman Chemistry, 254, 9291-9297 (1979)], or it is described that this reactive site is Arg-Met [see H. R. Lijnen et al.: Thrombosis Research, 39, 625-630 (1985)]. But its position has not yet been determined clearly. If the structures and positions of these plasmin binding sites and the reactive site and their vicinities are determined, it will be very beneficial and interesting to the utilization and development of human $\alpha_2$-plasmin inhibitor.

On the other hand, if a DNA of human $\alpha_2$-plasmin inhibitor or its fragment is obtained, it will become possible to produce part or the whole of the human $\alpha_2$-plasmin inhibitor by a gene manipulation technique. The utilization of such a gene or its fragment will permit diagnosis of human $\alpha_2$-plasmin inhibitor deficiency.

As a result of studies on human $\alpha_2$-plasmin inhibitor gene, the present inventors isolated complementary DNA (to be referred to as cDNA) of human $\alpha_2$-plasmin inhibitor containing 1209 nucleotides coding for the sequence of 403 amino acids from the carboxy-terminus of the human $\alpha_2$-plasmin inhibitor, and elucidated the detailed structure of the human $\alpha_2$-plasmin inhibitor on the carboxy-terminus side by analyzing the base sequence of the cDNA of human $\alpha_2$-plasmin inhibitor. This has led to the present invention.

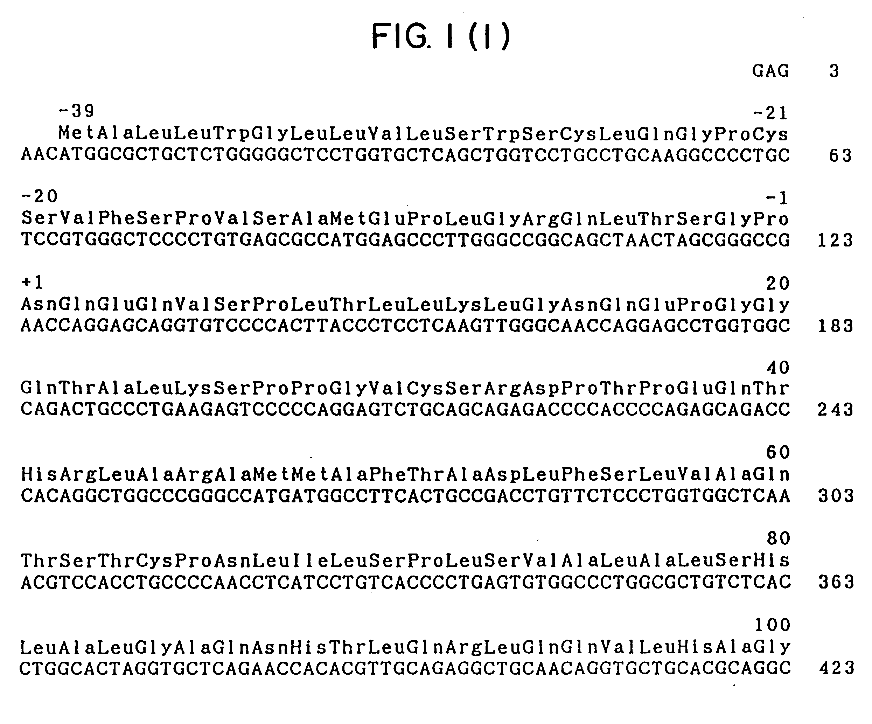

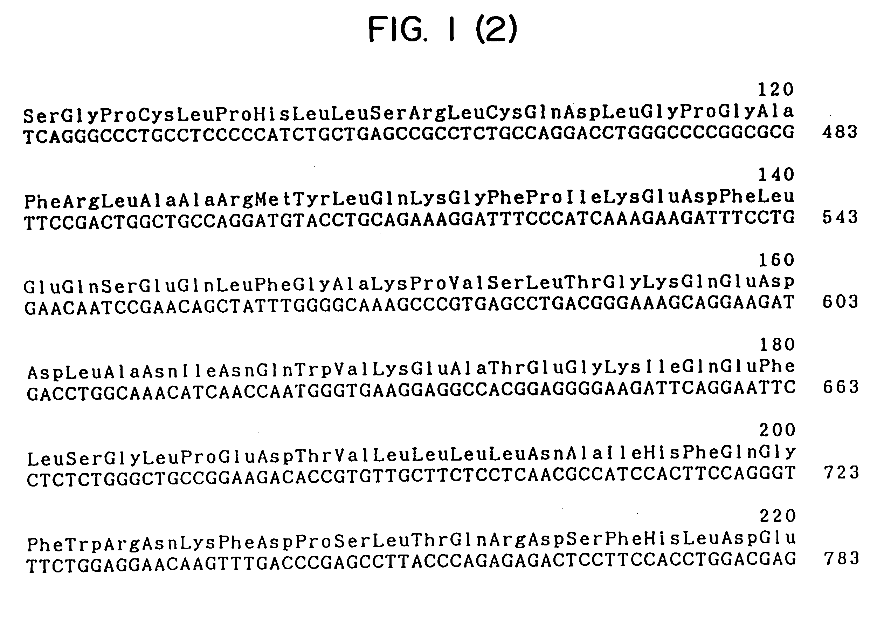

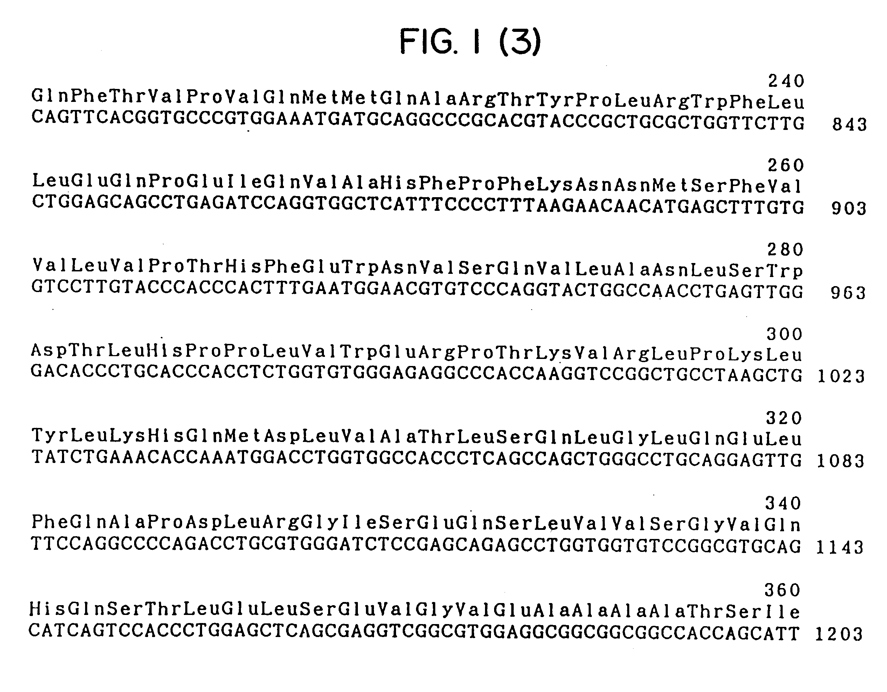

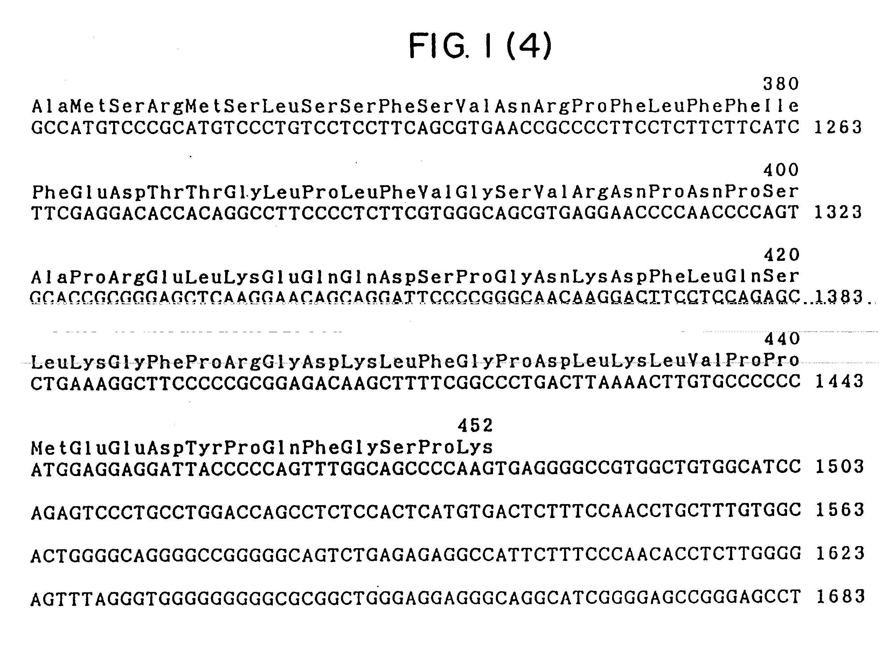

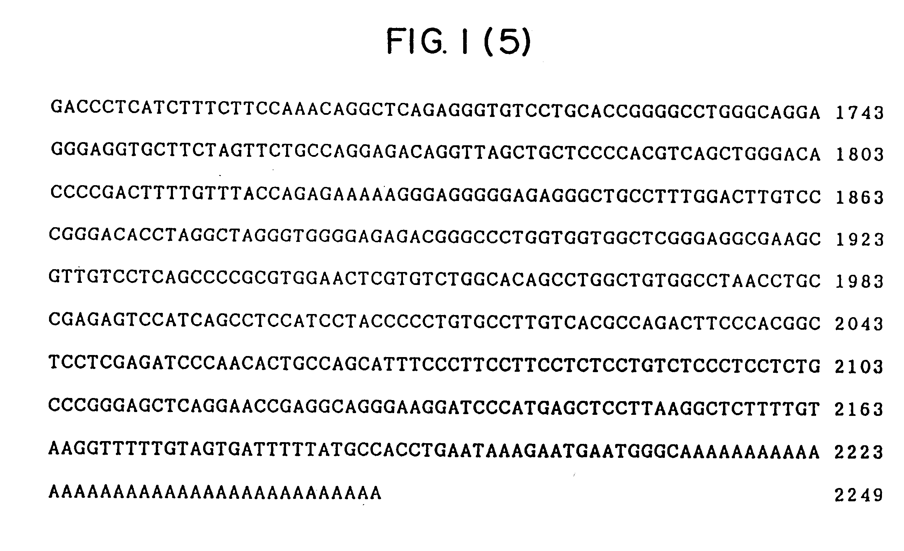

Specifically, according to one aspect of this invention, there is provided cDNA encoding a human $\alpha_2$-plasmin inhibitor precursor represented by the sequence of amino acids from the -39th Met to the 452nd Lys in Figure 1 of the accompanying drawings, preferably cDNA represented by the base sequence from the 7th A to the 1479th G in Figure 1. The invention also provides an amino acid sequence of a human $\alpha_2$-plasmin inhibitor precursor represented by the sequence of 491 amino acids from the -39th Met to the 452nd Lys in Figure 1.

In the amino acid sequence composed of 491 amino acids shown in Figure 1, the amino acid sequence from the 1st Asn to the 452nd Lys shows the protein of the human $\alpha_2$-plasmin inhibitor itself, and the amino acid sequence from the -39th Met to the -1st Pro shows the leader sequence.

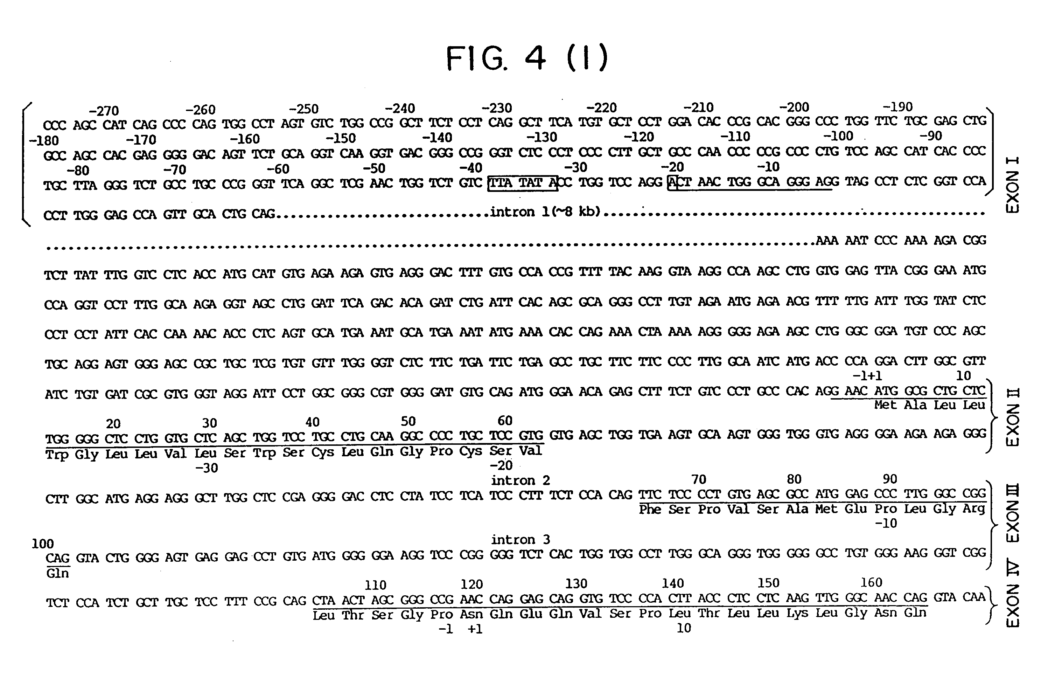

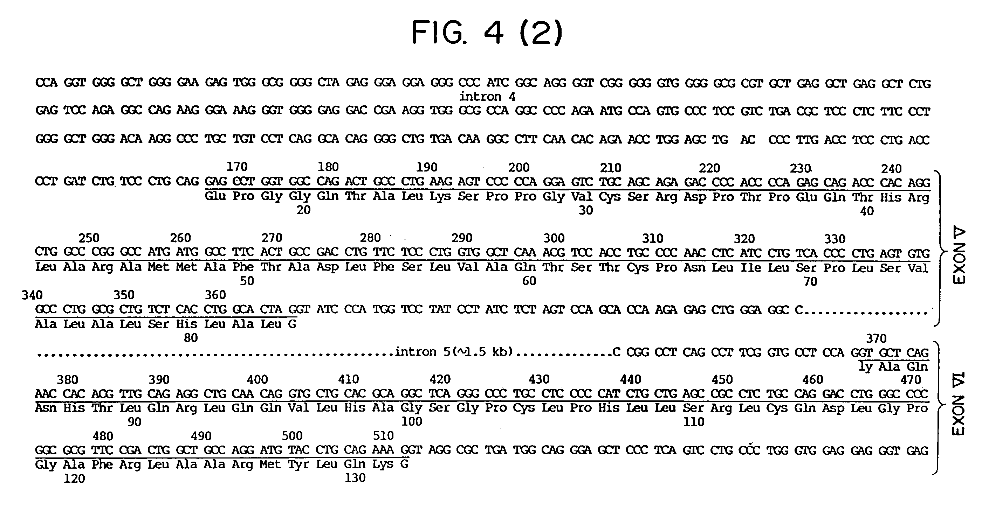

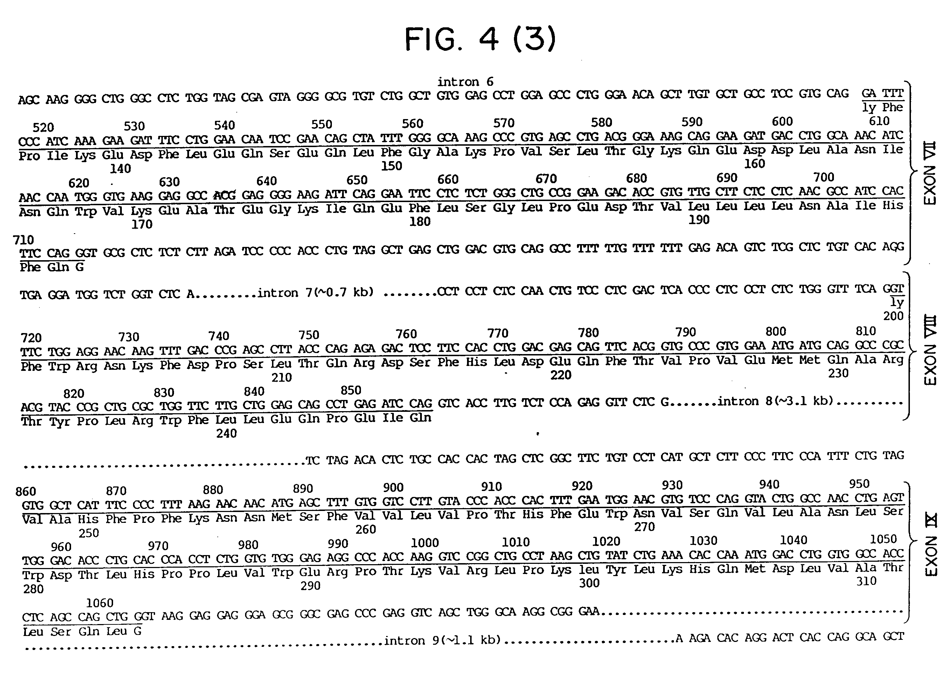

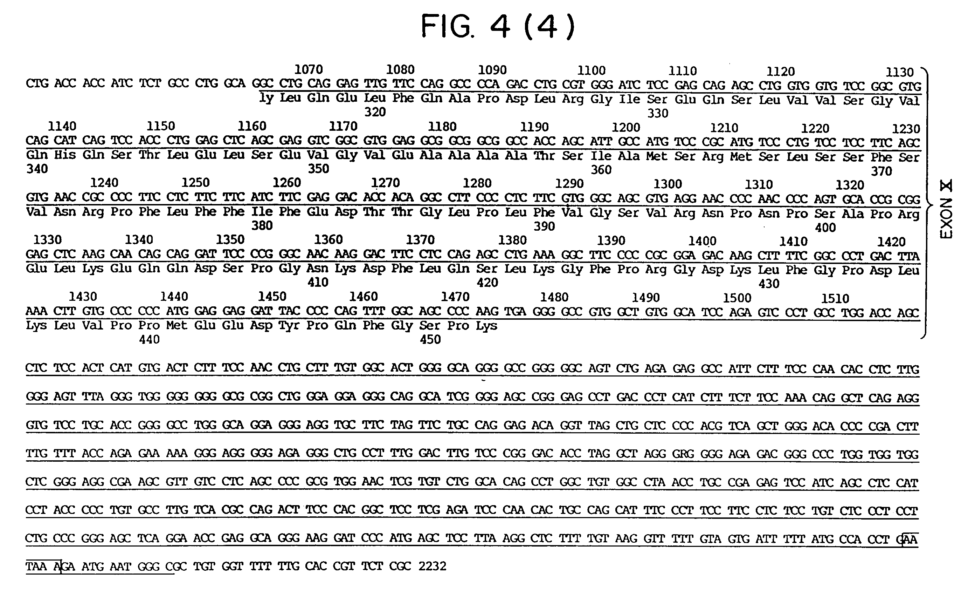

The present inventors furthered their studies on the elucidation of the structure of a genomic DNA coding for the human $\alpha_2$-plasmin inhibitor, and isolated human genomic DNAs coding for an amino acid sequence composed of 39 amino acids and corresponding to the propeptide of human $\alpha_2$-plasmin inhibitor and an amino acid sequence composed of 452 amino acids and constituting the human $\alpha_2$-plasmin inhibitor. Analysis of the base sequences of these amino acids has led to the discovery that the genomic DNA is a DNA composed of exons II, III, IV, V, VI, VII, VIII, IX and X, as shown in Figure 4, bonded to each other via introns and encoding human $\alpha_2$-plasmin inhibitor protein, and that exon I shown in Figure 4 is further bonded to the upstream end of exon II via an intron.

Thus, according to another aspect of this invention, there is provided a genomic DNA composed of 9 exons from exon II to X and 8 introns interposed between the adjoining exons, or a genomic gene consisting of the above genomic gene and an exon I bonded to the upstream end of the exon II via an intron.

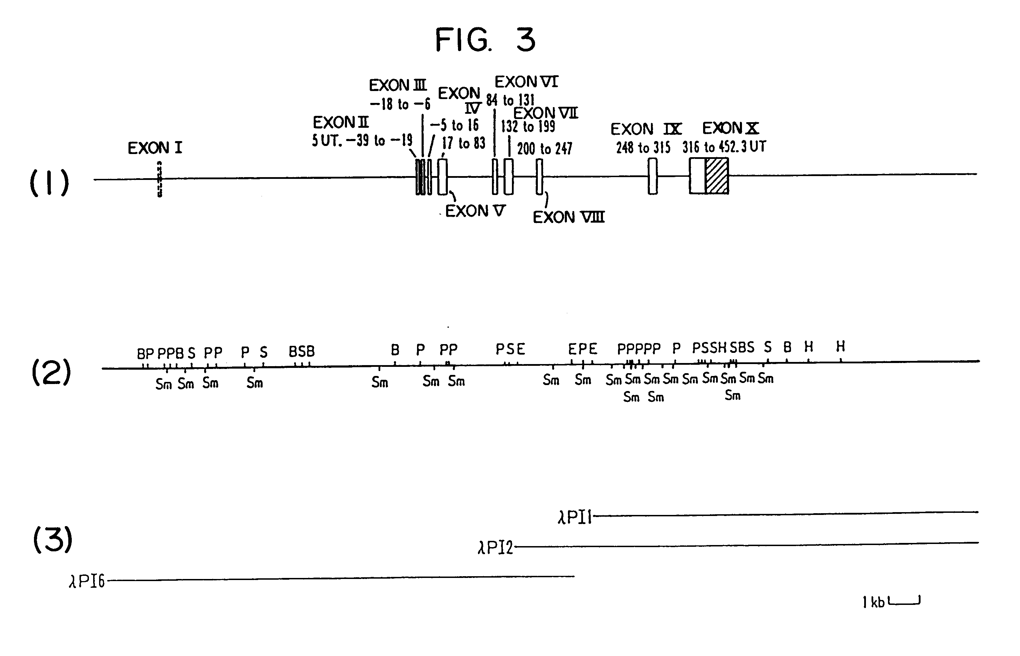

Part of exon II, exon III and part of exon IV in the genomic DNA of the invention encode a propeptide of 39 amino acids. The remaining part of exon IV, exon V, exon VI, exon VII, exon VIII, exon IX and part of exon X encode human $\alpha_2$-plasmin inhibitor. The restriction endonuclease map of the genomic DNA of the

human $\alpha_2$-plasmin inhibitor with regard to six restriction endonucleases is shown in Figure 3 (2). This genomic gene was obtained as clones of three lambda phages shown in Figure 3 (3).

The base sequence and amino acid sequence of the genomic DNA of human $\alpha_2$-plasmin inhibitor are shown in Figure 4. In Figure 4, the exons are the underlined portions of the base sequence. Exon I does not encode the amino acid sequence. Exon II consists of 67 bases in total, four bases (GAAC) in the non-coding region and 63 bases encoding 21 amino acids in part of the propeptide. Exon III consists of 39 bases encoding 13 amino acids in part of the propeptide. Exon IV consists of 63 bases in total, 15 bases encoding 5 amino acids in part of the propeptide and 48 based encoding 16 amino acids in the N-terminus portion of the $\alpha_2$-plasmin inhibitor. Exon V consists of 202 bases encoding 67 1/3 amino acids of $\alpha_2$-plasmin inhibitor. Exon VI consists of 144 bases encoding 48 amino acids of $\alpha_2$-plasmin inhibitor. Exon VII consists of 204 bases encoding 68 amino acids of $\alpha_2$-plasmin inhibitor. Exon VIII consists of 143 bases encoding 47 2/3 amino acids. of $\alpha_2$-plasmin inhibitor. Exon IX consists of 205 bases encoding 68 1/3 amino acids of $\alpha_2$-plasmin inhibitor. Exon X consists of 1160 bases in total, 410 bases encoding 136 2/3 amino acids on the C-terminus of $\alpha_2$-plasmin inhibitor and 759 bases in the non-coding region. The DNA base sequences of these exons are underlined.

The following Examples illustrate isolation of messenger RNA ("mRNA" for short) from human liver cells, construction of a cDNA library, screening of human $\alpha_2$-plasmin inhibitor, recloning of human $\alpha_2$-plasmin inhibitor cDNA, preparation of a restriction endonuclease cleavage map of human $\alpha_2$-plasmin inhibitor, determination of the base sequence of human $\alpha_2$-plasmin inhibitor cDNA, cloning of human $\alpha_2$-plasmin inhibitor genomic DNA, preparation of a restriction endonuclease map of the human $\alpha_2$-plasmin inhibitor genomic DNA, and determination of the base sequence of the human $\alpha_2$-plasmin inhibitor genomic DNA in detail.

It should be understood that in the present specification and the accompanying drawings, amino acids and polypeptides are abbreviated by the method of IUPACIUB, Commission on Biological Nomenclature (CBN). For example, the following abbreviations are used.

Ala: L-alanine
Arg: L-arginine
Asn: L-asparagine
Asp: L-aspartic acid
Cys: L-cysteine
Gln: L-glutamine
Glu: L-glutamic acid
Gly: glycine
His: L-histidine
Ile: L-isoleucine
Leu: L-leucine
Lys: L-lysine
Met: L-methionine
Phe: L-phenylalanine
Pro: L-proline
Ser: L-serine
Thr: L-threonine
Trp: L-tryptophan
Tyr: L-tyrosine
Val: L-valine

The DNA sequence will be shown in abbreviations by bases contained in the constituent deoxyribonucleotides. For example, the following abbreviations are used.

A: adenine (representing deoxyadenylic acid)
C: cytosine (representing deoxycytidylic acid)
G: guanine (representing deoxyguanylic acid)
T: thymine (representing deoxythymidylic acid)

Brief Description of the Accompanying Drawings

Figure 1 shows the amino acid sequence of human $\alpha_2$-plasmin inhibitor in this invention and a cDNA thereof.

Figure 2 is a restriction endonuclease map of the cDNA of human $\alpha_2$-plasmin inhibitor. The white portion is a coding region of the amino acid sequence of the invention and the black portion is a non-coding region.

Figure 3 (1) shows introns-exons of the genomic DNA of human $\alpha_2$-plasmin inhibitor. The ten exons are shown by rectangles. Exon I, part of exon II and part of exon X are non-coding regions which do not encode amino acids. The non-coding region of exon X is shown by hatchings.

Figure 3 (2) is a restriction endonuclease map of the genomic DNA of human $\alpha_2$-plasmin inhibitor in accordance with this invntion. The symbols show the following endonucleases.

B: BamHI
E: EcoRI
H: HindIII
P: PstI
S: SacI
Sm: SmaI

Figure 3 (3) shows DNA fragments in phage clones encoding the genomic DNA of human $\alpha_2$-plasmin inhibitor in accordance with this invention.

Figures 3 (1) to (3) correspond to each other vertically.

Figure 4 shows the DNA base sequence and amino acid sequence of the genomic DNA of human $\alpha_2$-plasmin inhibitor in accordance with this invention. The exon portions are underlined, and the corresponding amino acids are indicated beneath. A sequence presuumably corresponding to the TATA box upstream of exon I and the transcriptional site are surrounded by lines. The polyadenylation recognition site downstream of 3′-terminus is surrounded by a line.

EXAMPLE 1

Isolation of mRNA from human liver cells

mRNA was isolated from human liver cells in accordance with the guanidine thiocyanate method [see J. M. Chirgwin et al.: Biochemistry, 18, 5294-5299 (1979)]. To 2 $\times$ $10^8$ human liver cells was added 5 ml of GTC solution (6M guanidine isothiocyanate, 5mM sodium citrate, 0.1M 20-mercaptoethanol, 0.5% sodium N-lauroylsarcosinate), and the mixture was homogenized. The homogenate was superimposed on 3.8 ml of an aqueous solution containing 5.7M CsCl and 0.1M EDTA. They were ultracentrifuged using an RPS-50T rotor (Hitachi) at 35,000 rpm for 15 hours. After ultracentrifugation, the solution was carefully removed, and the residue was rinsed three times with about 1 ml of ethanol, and dissolved in 1.4 ml of water. The solution was treated with ethanol to form a precipitate. The precipitate was dissolved in 0.5 ml of a washing solution composed of 0.5M NaCl, 18mM of Tris-HCl (pH 7.5), 1mM EDTA and 0.05% of SDS, and the solution was passed through 0.5 ml of Oligo(dT) cellulose column. The column was washed with the above washing solution, and eluted with an eluent composed of 10mM Tris-HCl (pH 7.5), 1mM EDTA and 0.05% SDS to give about 31 micrograms of polyA+ mRNA.

EXAMPLE 2

Construction of cDNA library

cDNA was synthesized from the polyA+ mRNA derived from human liver cells by the method of Gubler and Hoffman [see U.Gubler & B. J. Hoffman: Gene, 25, 263-269 (1983)] using a cDNA synthesis kit made by Amersham Co.

A single-stranded cDNA was synthesized in a yield of about 30% by adding 5 micrograms of Oligo(dT) 12-18 to 5 micrograms of polyA+ mRNA in the presence of 50 units of RNase inhibiting enzyme (HPRI) derived from human placenta and causing transcriptase to act at 42°C for 1.5 hours. Four units of E. coli ribonuclease H and 115 units of E. coli DNA polymerase I were added to the resulting reaction solution, and reacted at 12°C for 1 hour and then at 22°C for 1 hour. The reaction mixture was left to stand at 70°C for 10 minutes to deactivate the enzymes. Then, 10 units of T4-DNA polymerase was added and reacted at 47°C for 10 minuts to obtain a double-stranded cDNA in a yield of about 95%. The double-stranded cDNA was reacted with 20 units of EcoRI methylase at 37°C for 1 hour. To the reaction product was bonded 16 units of EcoRI linker (made by Takara Shuzo). Then, 16 units of EcoRI (made by Takara Shuzo) was added and the mixture was reacted at 37°C for 2 hours. The reaction mixture was passed through a column of Sepharose CL-4B to purify it and 0.34 microgram of cDNA was obtained. This cDNA (0.4 microgram) and 1.0 microgram of λgt10 arm (made by Vector Cloning Systems) were ligated to obtain a hybrid DNA having the cDNA derived from human liver cells inserted into it. The resulting hybrid DNA was packaged in vitro -

[see A. Becker & M. Gold: P Natl. Acad. Sci. USA, 72, 581 (1975)] to obtain a cDNA library derived from human liver cells.

EXAMPLE 3

Screening of human α 2-plasmin inhibitor cDNA

The library of cDNA derived from human liver cells obtained in Example 2 was transfected into E. coli C 600 hfl⁻ strain to form plaques. Clones containing human α 2-plasmin inhibitor DNA were selected in accordance with the Benton and Davis plaque hybridization method using synthetic DNAs P-1 and P-2 labelled with [$^{32}$P] [see W. D. Benton & R. W. Davis: Science, 196, 180 (1977)]. The synthetic DNAs used as probes were synthesized by using a DNA sequence corresponding to the partial amino acid sequence of human α 2-plasmin inhibitor reported by Collen et al. [see D. Collen et al.: Thrombosis and Haemostasis, 48, 311-314 (1982)] as a DNA synthesizer (made by Applied Biosystems).

```
(P-1)  Met Glu Glu Asp Tyr Pro
   3'  TAC CTT CTT CTG ATG GG   5'
             C   C   A   A

(P-2)  Val Glu Met Met Gln Ala
   3'  CAG TCT TAC TAC GTT CG   5'
         A   C           C
         T
         C
```

EXAMPLE 4

Recloning of human α2-plasmin inhibitor cDNA

Plasmid pUC8 (2 micrograms) was digested with restriction endonuclease EcoRI in accordance with Example 2, and 1.9 units of alkaline phosphatase (E. coli C75; a product of Takara Shuzo) was added. The mixture was reacted at 58 ° C for 2 hours. After the reaction, the reaction solution was extracted with phenol three times to deactivate and remove the alkaline phosphatase in the reaction solution. The EcoRI-digested cDNA fragment of human α2-plasmin inhibitor obtained in Example 3 was added to the resulting EcoRI/alkaline phosphatase treated solution of pUC8, and 2 units of T4-DNA ligase was reacted with the mixture at 12 ° C for 16 hours to perform ligation.

E. coli LE 392 was transformed. with hybrid DNA prepared by ligating the EcoRI-digested cDNA fragment of human α2-plasmin inhibitor in accordance with an ordinary $CaCl_2$ method [see. M. V. Norgard et al.: Gene, 3, 297 (1978)]. The transformants were inoculated in L-broth plates containing ampicillin in a concentration of 50 micrograms/ml. The plates were cultivated overnight at 37 ° C to grow the transformants. DNAs were prepared from the resulting colonies by a known method, and by agarose gel electrophoresis, the desired hybrid DNAs were determined. They were named pPI 41, pPI 39 and pPI 142.

EXAMPLE 5

Preparation of a restriction endonuclease map of human α2-plasmin inhibitor cDNA

Lambda gt10DNA containing the human α2-plasmin inhibitor cDNA obtained in Example 3 was digested with EcoRI to cut out the inserted human α2-plasmin inhibitor cDNA and isolated by 0.8% agarose gel electrophoresis. This human α2-plasmin inhibitor cDNA fragment (0.1 microgram) was dissolved in 10 microliters of a buffer for restriction endonucleases [an aqueous solution containing 100mM NaCl, 50mM Tris-HCl (pH 7.5), 10mM $MgCl_2$ and 1mM dithiothreitol for digestion with EcoRI; an aqueous solution containing 50mM NaCl, 10mM Tris-HCl (pH 7.5), 10mM $MgCl_2$ and 1mM dithiothreitol for digestion with BamHI, PstI and HindIII; and an aqueous solution containing 20mM KCl, 10mM Tris-HCl (pH 8.0), 10mM $MgCl_2$ and 1mM dithiothreitol for digestion with SmaI, respectively], and digested at 37°C for 1 hour. When the digestion was carried out using two restriction endonucleases, the cDNA fragment was first treated with one restriction endonuclease which acted at a low salt concentration. Then, the salt concentration was raised to a predetermined concentration, and the DNA fragment was treated with the other enzyme which acted at a high salt concentration.

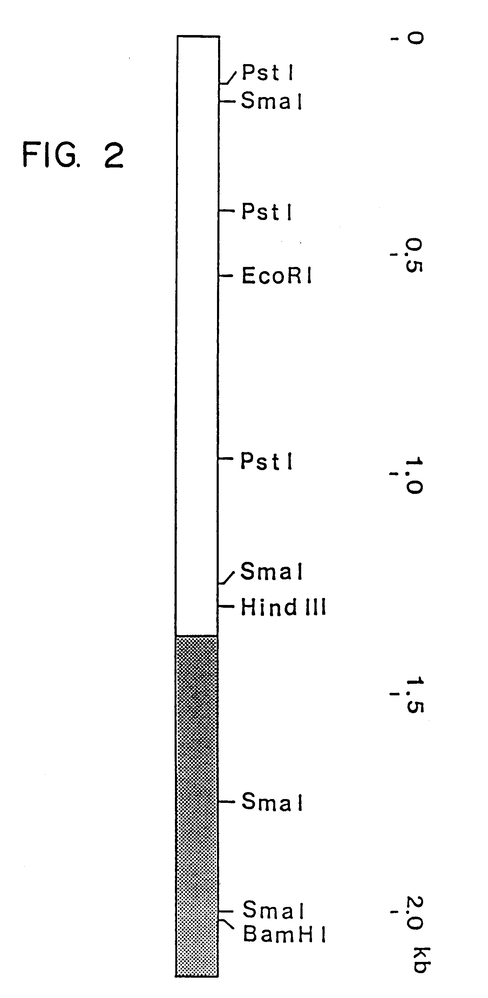

After digestion, 1 microliter of 0.25% bromophenol blue and a 50% aqueous solution of glycerol were added, and the mixture was subjected to electrophoresis using 0.8%-1.2% agarose containing 1 microgram/ml of ethidium bromide. At the time of electrophoresis, a digestion product of the DNA of lambda phage with HindIII was used as a molecular size marker for the DNA fragment. After the electrophoresis, ultraviolet light was irradiated on the gel, and the digestion pattern was observed. The patterns of digestions with various restriction endonucleases alone or combinations of two restriction endonucleases were analyzed. The restriction endonuclease map thus obtained of the human $\alpha_2$-plasmin inhibitor cDNA having a molecular size of about 2.2 Kb is shown in Figure 2.

EXAMPLE 6

Determination of the base sequence of human $\alpha_2$-plasmin inhibitor cDNA

The base sequence of the human $\alpha_2$-plasmin inhibitor cDNA was determined by the Sanger's dideoxy sequence method [see F. Sanger et al.: Proc. Natl. Acad. Sci. USA, 74, 5463-5467 (1977)].

For example, The EcoRI fragment of the human $\alpha_2$-plasmin inhibitor cDNA was inserted into the EcoRI site of M13 mp18 or M13 mp19 vector, and E. coli JM105 strain was transformed with the vector. A single-stranded hybrid DNA was prepared by a known method using the transformed JM105. The single-stranded DNA was subjected to sequence reaction using an M13 sequence kit (made by Amersham), and then subjected to electrophoresis using 6% polyacrylamide gel containing 7M urea. The gel was radioautographed overnight at -80°C. The separation pattern was analyzed to obtain data for determination of the base sequence of human $\alpha_2$-plasmin inhibitor.

As a result, the base sequence encoding the human $\alpha_2$-plasmin inhibitor was determined, and is shown in Figure 1.

EXAMPLE 7

Cloning of human $\alpha_2$-plasmin inhibitor genomic DNA

DNA extracted from human placenta was digested with restriction endonucleases AluI and HaeIII, and inserted into Charon 4A bacteriophage vector arm via an EcoRi linker to construct a library. The plaques ($1.2 \times 10^6$) of the library were screened using cDNA fragments corresponding to an amino acid sequence from the 31st to the 130th amino acids from the N-terminus of the amino acid sequence of $\alpha_2$-plasmin inhibitor and a cDNA fragment corresponding to the 179th to the 429th amino acids from the N-terminus of the amino acid sequence of $\alpha_2$-plasmin inhibitor as probes [see W. D. Benton & R. W. Davis: Science, 196, 180-182 (1977)]. For the screening of clones upstream of 3′, a synthetic DNA (5′ACTCCCCTGCCAGCC3′) composed of 15 bases was used as a probe. When cDNAs were used as probes, the cDNA fragments were labelled with $^{32}P$ by nick translation. The synthetic DNA was labelled on the 5′ side with T4 polynucleotidekinase.

As a result of screening, three clones, λPI1, λPI2 and λPI6, encoding the entire region of the genomic DNA of $\alpha_2$-plasmin inhibitor were obtained. The correspondence of the clones to the genomic DNA is shown in Figures 3 (1), (2) and (3).

EXAMPLE 8

Preparation of a restriction endonuclease cleavage map of human $\alpha_2$-plasmin inhibitor genomic DNA:-

The phage DNA containing human $\alpha_2$-plasmin inhibitor genomic DNA obtained in Example 7 was digested with EcoRI, and the inserted human $\alpha_2$-plasmin inhibitor genomic DNA was cut out and isolated by 0.9-1.5% agarose gel electrophoresis. This human $\alpha_2$-plasmin inhibitor DNA fragment (0.1 to 0.5 microgram) was dissolved in 10 microliters of a buffer for restriction endonucleases and digested with 2 units of various restriction endonucleases at 37°C for 1 hour.

When digestion was carried out using two restriction endonucleases, the DNA fragment was first treated with one restriction endonuclease which acted at a low salt concentration. Then, the salt concentration was raised to a predetermined concentration, and the DNA fragment was treated with the other restriction endonuclease which acted at a high salt concentration.

After digestion, 1 microliter of 0.25% bromophenol blue and a 50% aqueous solution of glycerol were added, and the mixture was subjected to electrophoresis using 0.8%-1.2% agarose containing 1 microgram/ml of ethidium bromide. At the time of electrophoresis, a digestion product of the DNA of lambda phage with HindIII was used as a molecular size marker for the DNA fragment. After the electrophoresis, ultraviolet light was irradiated on the gel, and the digestion pattern was observed. The patterns of digestions with various restriction endonucleases alone or combinations of two restriction were analyzed. The resulting restriction endonuclease map of human $\alpha_2$-plasmin inhibitor genomic DNA so obtained is shown in Figure 3 (2).

EXAMPLE 9

Determination of the base sequence of human $\alpha_2$-plasmin inhibitor genomic DNA

The base sequence of the human $\alpha_2$-plasmin inhibitor genomic DNA was determined by the Sanger's dideoxy sequence method [see F. Sanger et al.: Proc. Natl. Acad. Sci. USA, 74, 5463-5467 (1977)].

For example, The EcoRI fragment of the human $\alpha_2$-plasmin inhibitor genomic DNA was inserted into the EcoRI site of M13 mp18 or M13 mp19 vector, and E. coli JM105 strain was transformed with the vector. A single-stranded hybrid DNA was prepared by a known method using the transformed JM105. The single-stranded DNA was subjected to sequence reaction using an M13 sequence kit (made by Amersham), and then subjected to electrophoresis using 6% polyacrylamide gel containing 7M urea. The gel was radioautographed overnight at -80°C. The separation pattern was analyzed to obtain data for determination of the base sequence of human $\alpha_2$-plasmin inhibitor.

As a result, the base sequence of the genomic DNA encoding the human $\alpha_2$-plasmin inhibitor was determined, and is shown in Figure 4.

## Claims

1. cDNA encoding a human $\alpha_2$-plasmin inhibitor precursor protein represented by an amino acid sequence from the -39th Met to the 452nd Lys in Figure 1 of the accompanying drawings.

2. The cDNA of claim 1 which is represented by a base sequence from the 7th A to the 1479th G in Figure 1 of the accompanying drawings.

3. An amino acid sequence of a human $\alpha_2$-plasmin inhibitor precursor represented by an amino acid sequence from the -39th Met to the 452nd Lys in Figure 1 of the accompanying drawings.

4. Genomic DNA encoding a human $\alpha_2$-plasmin inhibitor protein which is composed of exons II, III, IV, V, VI, VII, VIII, IX and X in Figure 4 of the accompanying drawings, said exons being bonded to one another via introns.

5. The genomic DNA wherein exon I in Figure 4 is further bonded to exon II at its upstream end via an intron.

## Revendications

1. ADNc codant pour une protéine qui est un précurseur d'antiplasmine $\alpha_2$ humaine représenté par la séquence d'acides aminés allant du Met de la position -39 au Lys de la position 452 de la Figure 1 des dessins annexés.

2. ADNc selon la revendication 1, qui est représenté par une séquence de bases allant du A de la position 7 au G de la position 1479 de la Figure 1 des dessins annexés.

3. Séquence d'acides aminés d'un précurseur d'antiplasmine $\alpha_2$ humaine représenté par une séquence d'acides aminés allant du Met de la position -39 au Lys de la position 452 de la Figure 1 des dessins annexés.

4. ADN génomique codant pour une protéine d'antiplasmine $\alpha_2$ humaine, qui est composé des exons II, III, IV, V, VI, VII, VIII, IX et X de la Figure 4 des dessins annexés, lesdits exons étant reliés entre eux par des introns.

5. ADN génomique de la revendication 4, dans lequel l'exon I de la Figure 4 est de plus relié à l'exon II à l'extrémité amont de celui-ci par l'intermédiaire d'un intron.

## Patentansprüche

1. cDNA codierend für ein Human-$\alpha_2$-Plasmininhibitor-Vorläuferprotein, wiedergegeben durch eine Aminosäuresequenz von Met -39 bis Lys 452 in Fig. 1 der beigefügten Zeichnungen.

2. cDNA nach Anspruch 1, dadurch **gekennzeichnet,** daß sie durch eine Basensequenz von A 7 bis G 1479 in Fig. 1 der beigefügten Zeichnungen wiedergegeben ist.

3. Aminosäuresequenz eines Human-$\alpha_2$-Plasmininhibitor-Vorläufers, wiedergegeben durch eine Aminosäuresequenz von Met -39 bis Lys 452 in Fig. 1 der beigefügten Zeichnungen.

4. Genomische DNA codierend für ein Human-$\alpha_2$-Plasmiriinhibitorprotein,das aus den Exons II, III, IV, V, VI, VII, VIII, IX und X in Fig. 4 der beigefügten Zeichnungen zusammengesetzt ist, wobei die Exons aneinander über Introns gebunden sind.

5. Genomische DNA, dadurch **gekennzeichnet,** daß Exon I in Fig. 4 weiterhin an Exon II an dessen Stromaufwärts-Ende über ein Intron gebunden ist.

# FIG. 1 (1)

```
                                                            GAG    3

   -39                                                      -21
   MetAlaLeuLeuTrpGlyLeuLeuValLeuSerTrpSerCysLeuGlnGlyProCys
AACATGGCGCTGCTCTGGGGGCTCCTGGTGCTCAGCTGGTCCTGCCTGCAAGGCCCCTGC    63

-20                                                          -1
SerValPheSerProValSerAlaMetGluProLeuGlyArgGlnLeuThrSerGlyPro
TCCGTGGGCTCCCCTGTGAGCGCCATGGAGCCCTTGGGCCGGCAGCTAACTAGCGGGCCG   123

+1                                                           20
AsnGlnGluGlnValSerProLeuThrLeuLeuLysLeuGlyAsnGlnGluProGlyGly
AACCAGGAGCAGGTGTCCCCACTTACCCTCCTCAAGTTGGGCAACCAGGAGCCTGGTGGC   183

                                                             40
GlnThrAlaLeuLysSerProProGlyValCysSerArgAspProThrProGluGlnThr
CAGACTGCCCTGAAGAGTCCCCCAGGAGTCTGCAGCAGAGACCCCACCCCAGAGCAGACC   243

                                                             60
HisArgLeuAlaArgAlaMetMetAlaPheThrAlaAspLeuPheSerLeuValAlaGln
CACAGGCTGGCCCGGGCCATGATGGCCTTCACTGCCGACCTGTTCTCCCTGGTGGCTCAA   303

                                                             80
ThrSerThrCysProAsnLeuIleLeuSerProLeuSerValAlaLeuAlaLeuSerHis
ACGTCCACCTGCCCCAACCTCATCCTGTCACCCCTGAGTGTGGCCCTGGCGCTGTCTCAC   363

                                                            100
LeuAlaLeuGlyAlaGlnAsnHisThrLeuGlnArgLeuGlnGlnValLeuHisAlaGly
CTGGCACTAGGTGCTCAGAACCACACGTTGCAGAGGCTGCAACAGGTGCTGCACGCAGGC   423
```

# FIG. 1 (2)

```
                                                           120
SerGlyProCysLeuProHisLeuLeuSerArgLeuCysGlnAspLeuGlyProGlyAla
TCAGGGCCCTGCCTCCCCCATCTGCTGAGCCGCCTCTGCCAGGACCTGGGCCCCGGCGCG   483

                                                           140
PheArgLeuAlaAlaArgMetTyrLeuGlnLysGlyPheProIleLysGluAspPheLeu
TTCCGACTGGCTGCCAGGATGTACCTGCAGAAAGGATTTCCCATCAAAGAAGATTTCCTG   543

                                                           160
GluGlnSerGluGlnLeuPheGlyAlaLysProValSerLeuThrGlyLysGlnGluAsp
GAACAATCCGAACAGCTATTTGGGGCAAAGCCCGTGAGCCTGACGGGAAAGCAGGAAGAT   603

                                                           180
AspLeuAlaAsnIleAsnGlnTrpValLysGluAlaThrGluGlyLysIleGlnGluPhe
GACCTGGCAAACATCAACCAATGGGTGAAGGAGGCCACGGAGGGGAAGATTCAGGAATTC   663

                                                           200
LeuSerGlyLeuProGluAspThrValLeuLeuLeuLeuAsnAlaIleHisPheGlnGly
CTCTCTGGGCTGCCGGAAGACACCGTGTTGCTTCTCCTCAACGCCATCCACTTCCAGGGT   723

                                                           220
PheTrpArgAsnLysPheAspProSerLeuThrGlnArgAspSerPheHisLeuAspGlu
TTCTGGAGGAACAAGTTTGACCCGAGCCTTACCCAGAGAGACTCCTTCCACCTGGACGAG   783
```

# FIG. 1 (3)

```
                                                            240
GlnPheThrValProValGlnMetMetGlnAlaArgThrTyrProLeuArgTrpPheLeu
CAGTTCACGGTGCCCGTGGAAATGATGCAGGCCCGCACGTACCCGCTGCGCTGGTTCTTG   843

                                                            260
LeuGluGlnProGluIleGlnValAlaHisPheProPheLysAsnAsnMetSerPheVal
CTGGAGCAGCCTGAGATCCAGGTGGCTCATTTCCCCTTTAAGAACAACATGAGCTTTGTG   903

                                                            280
ValLeuValProThrHisPheGluTrpAsnValSerGlnValLeuAlaAsnLeuSerTrp
GTCCTTGTACCCACCCACTTTGAATGGAACGTGTCCCAGGTACTGGCCAACCTGAGTTGG   963

                                                            300
AspThrLeuHisProProLeuValTrpGluArgProThrLysValArgLeuProLysLeu
GACACCCTGCACCCACCTCTGGTGTGGGAGAGGCCCACCAAGGTCCGGCTGCCTAAGCTG  1023

                                                            320
TyrLeuLysHisGlnMetAspLeuValAlaThrLeuSerGlnLeuGlyLeuGlnGluLeu
TATCTGAAACACCAAATGGACCTGGTGGCCACCCTCAGCCAGCTGGGCCTGCAGGAGTTG  1083

                                                            340
PheGlnAlaProAspLeuArgGlyIleSerGluGlnSerLeuValValSerGlyValGln
TTCCAGGCCCCAGACCTGCGTGGGATCTCCGAGCAGAGCCTGGTGGTGTCCGGCGTGCAG  1143

                                                            360
HisGlnSerThrLeuGluLeuSerGluValGlyValGluAlaAlaAlaThrSerIle
CATCAGTCCACCCTGGAGCTCAGCGAGGTCGGCGTGGAGGCGGCGGCCACCAGCATT  1203
```

# FIG. 1 (4)

```
                                                                380
AlaMetSerArgMetSerLeuSerSerPheSerValAsnArgProPheLeuPhePheIle
GCCATGTCCCGCATGTCCCTGTCCTCCTTCAGCGTGAACCGCCCCTTCCTCTTCTTCATC 1263

                                                                400
PheGluAspThrThrGlyLeuProLeuPheValGlySerValArgAsnProAsnProSer
TTCGAGGACACCACAGGCCTTCCCCTCTTCGTGGGCAGCGTGAGGAACCCCAACCCCAGT 1323

                                                                420
AlaProArgGluLeuLysGluGlnGlnAspSerProGlyAsnLysAspPheLeuGlnSer
GCACCGCGGGAGCTCAAGGAACAGCAGGATTCCCCGGGCAACAAGGACTTCCTCCAGAGC 1383

                                                                440
LeuLysGlyPheProArgGlyAspLysLeuPheGlyProAspLeuLysLeuValProPro
CTGAAAGGCTTCCCCCGCGGAGACAAGCTTTTCGGCCCTGACTTAAAACTTGTGCCCCCC 1443

                                    452
MetGluGluAspTyrProGlnPheGlySerProLys
ATGGAGGAGGATTACCCCCAGTTTGGCAGCCCCAAGTGAGGGGCCGTGGCTGTGGCATCC 1503

AGAGTCCCTGCCTGGACCAGCCTCTCCACTCATGTGACTCTTTCCAACCTGCTTTGTGGC 1563

ACTGGGGCAGGGGCCGGGGGCAGTCTGAGAGAGGCCATTCTTTCCCAACACCTCTTGGGG 1623

AGTTTAGGGTGGGGGGGGGCGCGGCTGGGAGGAGGGCAGGCATCGGGGAGCCGGGAGCCT 1683
```

FIG. 1 (5)

```
GACCCTCATCTTTCTTCCAAACAGGCTCAGAGGGTGTCCTGCACCGGGGCCTGGGCAGGA 1743
GGGAGGTGCTTCTAGTTCTGCCAGGAGACAGGTTAGCTGCTCCCCACGTCAGCTGGGACA 1803
CCCCGACTTTTGTTTACCAGAGAAAAAGGGAGGGGGAGAGGGCTGCCTTTGGACTTGTCC 1863
CGGGACACCTAGGCTAGGGTGGGGAGAGACGGGCCCTGGTGGTGGCTCGGGAGGCGAAGC 1923
GTTGTCCTCAGCCCCGCGTGGAACTCGTGTCTGGCACAGCCTGGCTGTGGCCTAACCTGC 1983
CGAGAGTCCATCAGCCTCCATCCTACCCCCTGTGCCTTGTCACGCCAGACTTCCCACGGC 2043
TCCTCGAGATCCCAACACTGCCAGCATTTCCCTTCCTTCCTCTCCTGTCTCCCTCCTCTG 2103
CCCGGGAGCTCAGGAACCGAGGCAGGGAAGGATCCCATGAGCTCCTTAAGGCTCTTTTGT 2163
AAGGTTTTTGTAGTGATTTTTATGCCACCTGAATAAAGAATGAATGGGCAAAAAAAAAAA 2223
AAAAAAAAAAAAAAAAAAAAAAAAAA                                   2249
```

FIG. 2

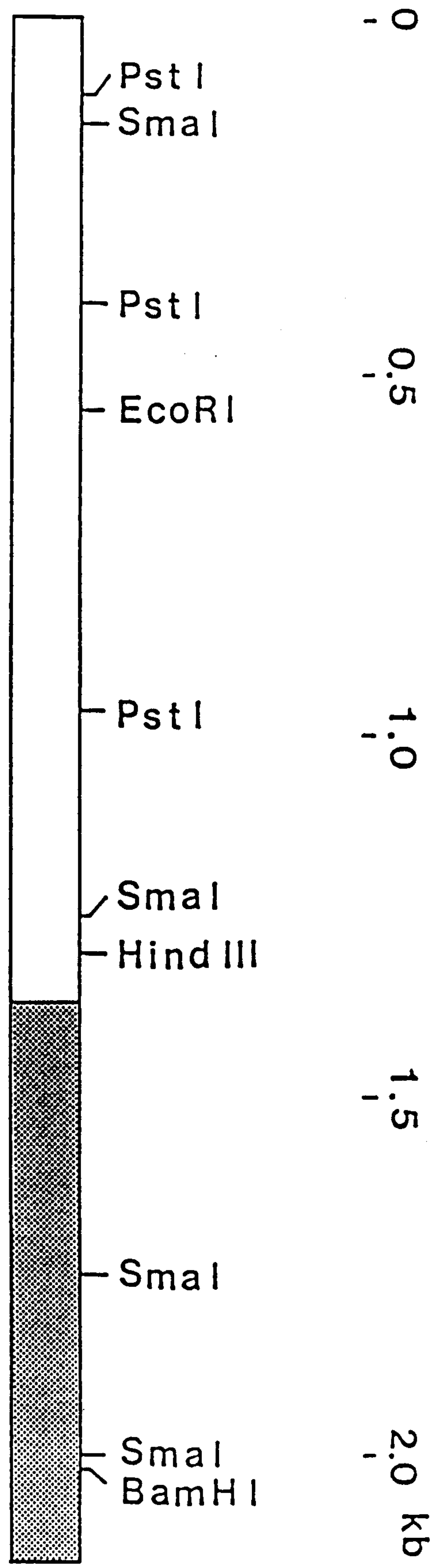
Pst I
Sma I
Pst I
EcoR I
Pst I
Sma I
Hind III
Sma I
Sma I
BamH I
0
0.5
1.0
1.5
2.0 kb

FIG. 3

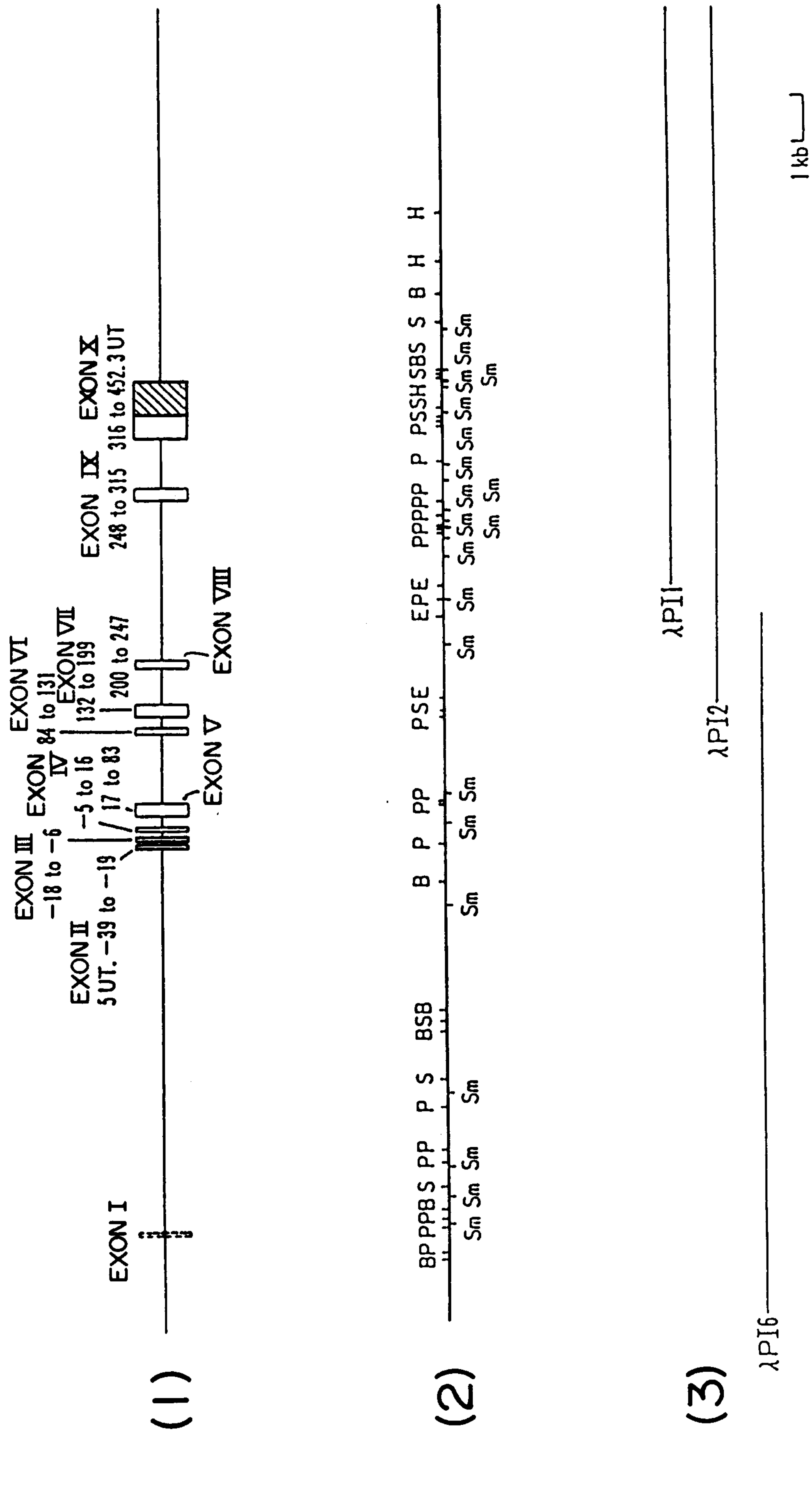
(1)
EXON I
EXON II
5 UT. −39 to −19
EXON III
−18 to −6
EXON IV
−5 to 16
17 to 83
EXON V
EXON VI
84 to 131
EXON VII
132 to 199
200 to 247
EXON VIII
EXON IX
248 to 315
EXON X
316 to 452.3 UT
(2)
BP PPB S PP P S BSB B P PP PSE EPE PPPPP P PSSHSBS S B H H
Sm Sm Sm Sm Sm Sm Sm Sm Sm Sm Sm Sm Sm Sm Sm Sm Sm Sm Sm Sm Sm Sm Sm Sm Sm Sm
(3)
λPI1
λPI2
λPI6
1 kb

# FIG. 4 (I)

EXON I

```
     -270      -260      -250      -240      -230      -220      -210      -200      -190
CCC AGC CAT CAG CCC CAG TGG CCT AGT GTC TGG CCG GCT TCT CCT CAG GCT TCA TGT GCT CCT GGA CAC CCG CAC GGG CCC TGG TTC TGC GAG CTG
-180      -170      -160      -150      -140      -130      -120      -110      -100      -90
GCC AGC CAC GAG GGG GAC AGT TCT GCA GGT CAA GGT GAC GGG CCG GGT CTC CCT CCC CTT GCT GCC CAA CCC CCG CCC CTG TCC AGC CAT CAC CCC
    -80       -70       -60       -50       -40       -30       -20       -10
TGC TTA GGG TCT GCC TGC CCG GGT TCA GGC TCG AAC TGG TCT GTC [TTA TAT A]CC TGG TCC AGG [A]CT AAC TGG GCA GGG AGG TAG CCT CTC GGT CCA

CCT TGG GAG CCA GTT GCA CTG CAG..........................intron 1(~8 kb)............................................

.........................................................................................AAA AAT CCC AAA AGA CGG

TCT TAT TTG GTC CTC ACC ATG CAT GTG AGA AGA GTG AGG GAC TTT GTG CCA CCG TTT TAC AAG GTA AGG CCA AGC CTG GTG GAG TTA CGG GAA ATG

CCA GGT CCT TTG GCA AGA GGT AGC CTG GAT TCA GAC ACA GAT CTG ATT CAC AGC GCA GGG CCT TGT AGA ATG AGA ACG TTT TTG ATT TGG TAT CTC

CCT CCT ATT CAC CAA AAC ACC CTC AGT GCA TGA AAT GCA TGA AAT ATG AAA CAC CAG AAA CTA AAA AGG GGG AGA AGC CTG GGC GGA TGT CCC AGC

TGC AGG AGT GGG AGC CGC TGC TCG TGT GTT TGG GGT CTC TTC TGA TTC TGA GCC TGC TTC TTC CCC TTG GCA ATC ATG ACC CCA GGA CTT GGC GTT
                                                                                                      -1+1            10
ATC TGT GAT CGC GTG GGT AGG ATT CCT GGC GGG CGT GGG GAT GTG CAG ATG GGA ACA GAG CTT TCT GTC CCT GCC CAC AGG AAC ATG GCG CTG CTC
                                                                                                          Met Ala Leu Leu
```

EXON II

```
        20        30        40        50        60
TGG GGG CTC CTG GTG CTC AGC TGG TCC TGC CTG CAA GGC CCC TGC TCC GTG GTG AGC TGG TGA AGT GCA AGT GGG TGG GTG AGG GGA AGA AGA GGG
Trp Gly Leu Leu Val Leu Ser Trp Ser Cys Leu Gln Gly Pro Cys Ser Val
                    -30                                 -20
```

EXON III

```
                                                      intron 2                70        80        90
CTT GGC ATG AGG AGG GCT TGG CTC CGA GGG GAC CTC CTA TCC TCA TCC CTT TCT CCA CAG TTC TCC CCT GTG AGC GCC ATG GAG CCC TTG GGC CGG
                                                                              Phe Ser Pro Val Ser Ala Met Glu Pro Leu Gly Arg
                                                                                                          -10
100                                                   intron 3
CAG GTA CTG GGG AGT GAG GAG CCT GTG ATG GGG GGA AGG TCC CGG GGG TCT CAC TGG TGG CCT TGG GCA GGG TGG GGG GCC TGT GGG AAG GGT CGG
Gln
```

EXON IV

```
                                    110       120       130       140       150       160
TCT CCA TCT GCT TGC TCC TTT CCG CAG CTA ACT AGC GGG CCG AAC CAG GAG CAG GTG TCC CCA CTT ACC CTC CTC AAG TTG GGC AAC CAG GTA CAA
                                    Leu Thr Ser Gly Pro Asn Gln Glu Gln Val Ser Pro Leu Thr Leu Leu Lys Leu Gly Asn Gln
                                                    -1  +1                        10
```

# FIG. 4 (2)

```
CCA GGT GGG GCT GGG GAA GAG TGG GCG GGG CTA GAG GGA GGA GGG CCC ATC GGC AGG GGT CGG GGG GTG GGG GCG CGT GCT GAG GCT GAG GCT CTG
                                                      intron 4
GAG TCC AGA GGC CAG AAG GGA AAG GGT GGG GAG GAC CGA AGG TGG GCG CCA GGC CCC AGA ATG CCA GTG CCC TCC GTC TGA CGC TCC CTC TTC CCT

GGG GCT GGG ACA AGG CCC TGC TGT CCT CAG GCA CAG GGG CTG TGA CAA GGC CTT CAA CAC AGA ACC TGG AGC TG  AC  CCC TTG ACC TCC CTG ACC

                               170         180         190         200         210         220         230         240
CCT GAT CTG TCC CTG CAG GAG CCT GGT GGC CAG ACT GCC CTG AAG AGT CCC CCA GGA GTC TGC AGC AGA GAC CCC ACC CCA GAG CAG ACC CAC AGG   } EXON V
                        Glu Pro Gly Gly Gln Thr Ala Leu Lys Ser Pro Pro Gly Val Cys Ser Arg Asp Pro Thr Pro Glu Gln Thr His Arg
                                     20                                      30                                      40

     250         260         270         280         290         300         310         320         330
CTG GCC CGG GCC ATG ATG GCC TTC ACT GCC GAC CTG TTC TCC CTG GTG GCT CAA ACG TCC ACC TGC CCC AAC CTC ATC CTG TCA CCC CTG AGT GTG
Leu Ala Arg Ala Met Met Ala Phe Thr Ala Asp Leu Phe Ser Leu Val Ala Gln Thr Ser Thr Cys Pro Asn Leu Ile Leu Ser Pro Leu Ser Val
                                 50                                      60                                      70
340         350         360
GCC CTG GCG CTG TCT CAC CTG GCA CTA GGT ATC CCA TGG TCC TAT CCT ATC TCT AGT CCA GCA CCA AGA GAG CTG GGA GGC C.................
Ala Leu Ala Leu Ser His Leu Ala Leu G
                         80                                                                                         370
..................................................intron 5(~1.5 kb)..............C CGG CCT CAG CCT TCG GTG CCT CCA GGT GCT CAG
                                                                                                                 ly Ala Gln
   380         390         400         410         420         430         440         450         460         470
AAC CAC ACG TTG CAG AGG CTG CAA CAG GTG CTG CAC GCA GGC TCA GGG CCC TGC CTC CCC CAT CTG CTG AGC CGC CTC TGC CAG GAC CTG GGC CCC   } EXON VI
Asn His Thr Leu Gln Arg Leu Gln Gln Val Leu His Ala Gly Ser Gly Pro Cys Leu Pro His Leu Leu Ser Arg Leu Cys Gln Asp Leu Gly Pro
             90                                      100                                     110
       480         490         500         510
GGC GCG TTC CGA CTG GCT GCC AGG ATG TAC CTG CAG AAA GGT AGG CGC TGA TGG CAG GGA GCT CCC TCA GTC CTG CCC TGG GTG GAG GAG GGT GAG
Gly Ala Phe Arg Leu Ala Ala Arg Met Tyr Leu Gln Lys G
    120                                     130
```

# FIG. 4 (3)

EXON VII

```
                                                  intron 6
AGC AAG GGG CTG GGC CTC TGG TAG CGA GTA GGG GCG TGT CTG GCT GTG GAG CCT GGA GCC CTG GGA ACA GCT TGT GCT GCC TCC GTG CAG  GA TTT
                                                                                                                          ly Phe
520         530         540         550         560         570         580         590         600         610
CCC ATC AAA GAA GAT TTC CTG GAA CAA TCC GAA CAG CTA TTT GGG GCA AAG CCC GTG AGC CTG ACG GGA AAG CAG GAA GAT GAC CTG GCA AAC ATC
Pro Ile Lys Glu Asp Phe Leu Glu Gln Ser Glu Gln Leu Phe Gly Ala Lys Pro Val Ser Leu Thr Gly Lys Gln Glu Asp Asp Leu Ala Asn Ile
            140                                     150                                         160
    620         630         640         650         660         670         680         690         700
AAC CAA TGG GTG AAG GAG GCC ACG GAG GGG AAG ATT CAG GAA TTC CTC TCT GGG CTG CCG GAA GAC ACC GTG TTG CTT CTC CTC AAC GCC ATC CAC
Asn Gln Trp Val Lys Glu Ala Thr Glu Gly Lys Ile Gln Glu Phe Leu Ser Gly Leu Pro Glu Asp Thr Val Leu Leu Leu Leu Asn Ala Ile His
                170                                     180                                     190
710
TTC CAG GGT GCG CTC TCT CTT AGA TCC CCC ACC CTG TAG GCT GAG CTG GAC GTG CAG GCC TTT TTG TTT TTT GAG ACA GTC TCG CTC TGT CAC AGG
Phe Gln G
```

EXON VIII

```
TGA GGA TGG TCT GGT CTC A.........intron 7(~0.7 kb) ........CCT CCT CTC CAA CTG TCC CTC GAC TCA CCC CTC CCT CTC TGG GTT TCA GGT
                                                                                                                              ly
                                                                                                                             200
720         730         740         750         760         770         780         790         800         810
TTC TGG AGG AAC AAG TTT GAC CCG AGC CTT ACC CAG AGA GAC TCC TTC CAC CTG GAC GAG CAG TTC ACG GTG CCC GTG GAA ATG ATG CAG GCC CGC
Phe Trp Arg Asn Lys Phe Asp Pro Ser Leu Thr Gln Arg Asp Ser Phe His Leu Asp Glu Gln Phe Thr Val Pro Val Glu Met Met Gln Ala Arg
                                    210                                 220                                         230
    820         830         840         850
ACG TAC CCG CTG CGC TGG TTC TTG CTG GAG CAG CCT GAG ATC CAG GTC ACC TTG TCT CCA GAG GTT CTC G.......intron 8(~3.1 kb).........
Thr Tyr Pro Leu Arg Trp Phe Leu Leu Glu Gln Pro Glu Ile Gln
                        240

.....................................TC TAG ACA CTC TGC CAC CAC TAG CTC GGC TTC TGT CCT CAT GCT CTT CCC TTC CCA TTT CTG TAG
```

EXON IX

```
860         870         880         890         900         910         920         930         940         950
GTG GCT CAT TTC CCC TTT AAG AAC AAC ATG AGC TTT GTG GTC CTT GTA CCC ACC CAC TTT GAA TGG AAC GTG TCC CAG GTA CTG GCC AAC CTG AGT
Val Ala His Phe Pro Phe Lys Asn Asn Met Ser Phe Val Val Leu Val Pro Thr His Phe Glu Trp Asn Val Ser Gln Val Leu Ala Asn Leu Ser
        250                                 260                                 270
    960         970         980         990         1000        1010        1020        1030        1040        1050
TGG GAC ACC CTG CAC CCA CCT CTG GTG TGG GAG AGG CCC ACC AAG GTC CGG CTG CCT AAG CTG TAT CTG AAA CAC CAA ATG GAC CTG GTG GCC ACC
Trp Asp Thr Leu His Pro Pro Leu Val Trp Glu Arg Pro Thr Lys Val Arg Leu Pro Lys leu Tyr Leu Lys His Gln Met Asp Leu Val Ala Thr
280                                     290                                 300                                         310
        1060
CTC AGC CAG CTG GGT AAG GAG GAG GGA GCG GGC GAG CCC GAG GTC AGC TGG GCA AGG CGG GAA......................................
Leu Ser Gln Leu G
...........................................intron 9(~1.1 kb)........................A AGA CAC AGG ACT CAC CAG GCA GCT
```

FIG. 4 (4)

```
                                    1070        1080        1090        1100        1110        1120        1130
CTG ACC ACC ATC TCT GCC CTG GCA GGC CTG CAG GAG TTG TTC CAG GCC CCA GAC CTG CGT GGG ATC TCC GAG CAG AGC CTG GTG GTG TCC GGC GTG
                                 ly Leu Gln Glu Leu Phe Gln Ala Pro Asp Leu Arg Gly Ile Ser Glu Gln Ser Leu Val Val Ser Gly Val
                                            320                                 330
   1140        1150        1160        1170        1180        1190        1200        1210        1220        1230
CAG CAT CAG TCC ACC CTG GAG CTC AGC GAG GTC GGC GTG GAG GCG GCG GCG GCC ACC AGC ATT GCC ATG TCC CGC ATG TCC CTG TCC TCC TTC AGC
Gln His Gln Ser Thr Leu Glu Leu Ser Glu Val Gly Val Glu Ala Ala Ala Ala Thr Ser Ile Ala Met Ser Arg Met Ser Leu Ser Ser Phe Ser
340                                     350                                 360                                     370
       1240        1250        1260        1270        1280        1290        1300        1310        1320
GTG AAC CGC CCC TTC CTC TTC TTC ATC TTC GAG GAC ACC ACA GGC CTT CCC CTC TTC GTG GGC AGC GTG AGG AAC CCC AAC CCC AGT GCA CCG CGG
Val Asn Arg Pro Phe Leu Phe Phe Ile Phe Glu Asp Thr Thr Gly Leu Pro Leu Phe Val Gly Ser Val Arg Asn Pro Asn Pro Ser Ala Pro Arg
                                380                                 390                                     400
 1330        1340        1350        1360        1370        1380        1390        1400        1410        1420
GAG CTC AAG CAA CAG CAG GAT TCC CCG GGC AAC AAG GAC TTC CTC CAG AGC CTG AAA GGC TTC CCC CGC GGA GAC AAG CTT TTC GGC CCT GAC TTA
Glu Leu Lys Glu Gln Gln Asp Ser Pro Gly Asn Lys Asp Phe Leu Gln Ser Leu Lys Gly Phe Pro Arg Gly Asp Lys Leu Phe Gly Pro Asp Leu
                                        410                             420                                 430
     1430        1440        1450        1460        1470        1480        1490        1500        1510
AAA CTT GTG CCC CCC ATG GAG GAG GAT TAC CCC CAG TTT GGC AGC CCC AAG TGA GGG GCC GTG GCT GTG GCA TCC AGA GTC CCT GCC TGG ACC AGC
Lys Leu Val Pro Pro Met Glu Glu Asp Tyr Pro Gln Phe Gly Ser Pro Lys
            440                                 450

CTC TCC ACT CAT GTG ACT CTT TCC AAC CTG CTT TGT GGC ACT GGG GCA GGG GCC GGG GGC AGT CTG AGA GAG GCC ATT CTT TCC CAA CAC CTC TTG

GGG AGT TTA GGG TGG GGG GGG GCG CGG CTG GGA GGA GGG CAG GCA TCG GGG AGC CGG GAG CCT GAC CCT CAT CTT TCT TCC AAA CAG GCT CAG AGG

GTG TCC TGC ACC GGG GCC TGG GCA GGA GGG AGG TGC TTC TAG TTC TGC CAG GAG ACA GGT TAG CTG CTC CCC ACG TCA GCT GGG ACA CCC CGA CTT

TTG TTT ACC AGA GAA AAA GGG AGG GGG AGA GGG CTG CCT TTG GAC TTG TCC CGG GAC ACC TAG GCT AGG GRG GGG AGA GAC GGG CCC TGG TGG TGG

CTC GGG AGG CGA AGC GTT GTC CTC AGC CCC GCG TGG AAC TCG TGT CTG GCA CAG CCT GGC TGT GGC CTA ACC TGC CGA GAG TCC ATC AGC CTC CAT

CCT ACC CCC TGT GCC TTG TCA CGC CAG ACT TCC CAC GGC TCC TCG AGA TCC CAA CAC TGC CAG CAT TTC CCT TCC TTC CTC TCC TGT CTC CCT CCT

CTG CCC GGG AGC TCA GGA ACC GAG GCA GGG AAG GAT CCC ATG AGC TCC TTA AGG CTC TTT TGT AAG GTT TTT GTA GTG ATT TTT ATG CCA CCT GAA

TAA AGA ATG AAT GGG CGC TGT GGT TTT TTG CAC CGT TCT CGC 2232
```

EXON X